# FASCICULE DE BREVET EUROPEEN

(11) **EP 1 144 019 B1**
(45) Date de publication et mention de la délivrance du brevet: **10.12.2003**
(21) Numéro de dépôt: 00900558.8
(22) Date de dépôt: 12.01.2000
(51) Int. Cl.: A61M 1/00, A61M 3/02

(54) **VAPORISATEUR CHIRURGICAL DE SERUM PHYSIOLOGIQUE**
CHIRURGISCHER VERNEBLER FÜR PHYSIOLOGISCHE LÖSUNGEN
SURGICAL SPRAYER OF PHYSIOLOGICAL SERUM

(30) Priorité: 13.01.1999 FR 9900608
(43) Date de publication de la demande: 17.10.2001
(73) Titulaire: Bartolome, Francis, 34150 Gignac (FR)
(72) Inventeur: Bartolome, Francis, 34150 Gignac (FR)
(86) Numéro de dépôt international: FR0000047
(87) Numéro de publication internationale: WO00041741

(56) Documents cités:
- DE-A- 19 644 979
- US-A- 4 108 178
- US-A- 4 372 307
- US-A- 4 648 838
- US-A- 4 696 669

## Description

L'invention est relative à un vaporisateur chirurgical de sérum physiologique, destiné à nettoyer le champ opératoire de toute présence de sang gênante pour le chirurgien, du genre comportant une canule constituée d'une première canalisation d'amenée d'air comprimé et d'une deuxième canalisation de sérum physiologique vers un embout distal servant de chambre de vaporisation et pourvu d'un orifice de sortie du mélange obtenu; la partie proximale de ladite canule étant reliée aux tuyaux d'amenée de l'air comprimé et du sérum en provenance d'une source située à distance.

L'état de la technique connue est essentiellement décrit dans les documents suivants :
- US-4108178 qui décrit un dispositif (utilisé en dentisterie) destiné à envoyer sous pression un jet d'eau dans la bouche;
- US-4372307 qui décrit un dispositif (utilisé en dentisterie) destiné à envoyer un mélange d'eau et d'air;
- US-4648838 qui décrit un dispositif (utilisé en dentisterie) destiné à envoyer de l'eau pourvu de moyens de réglage d'une source lumineuse;
- DE-19644979 qui décrit un dispositif à écrasement progressif simultané des canalisations d'eau et de liquide.

Le dispositif selon l'invention se caractérise en ce que :
- la jonction de la partie proximale de la canule, soit des canalisations qui la composent, et les tuyaux d'amenée de l'air comprimé et du sérum physiologique au vaporisateur proprement dit, est réalisée dans la zone d'une poignée creuse, dans laquelle passent lesdites canalisations et tuyaux, qui comporte deux moyens à écrasement progressif, indépendants, notamment du type roulettes de perfuseur, aptes à assurer le réglage, par le chirurgien lui-même, au cours de l'intervention, des débits d'air et de sérum;
- la poignée comporte, en combinaison, un moyen, du type vanne, agissant simultanément sur les passages d'air et de sérum de manière à fermer puis à ouvrir le passage de l'air et du sérum sans modifier les réglages préalablement établis par le chirurgien au moyen desdites roulettes.

Un tel vaporisateur améliore la qualité de l'intervention par son action dosée sur le champ opératoire, son adaptation au temps d'intervention, sa simplicité d'utilisation, sa bonne préhension (poignée adaptée) et sa compacité.

Les caractéristiques et les avantages de l'invention vont apparaître plus clairement à la lecture de la description détaillée qui suit d'au moins un mode de réalisation préféré de celle-ci donné à titre d'exemple non limitatif et représenté aux dessins annexés.

Sur ces dessins :

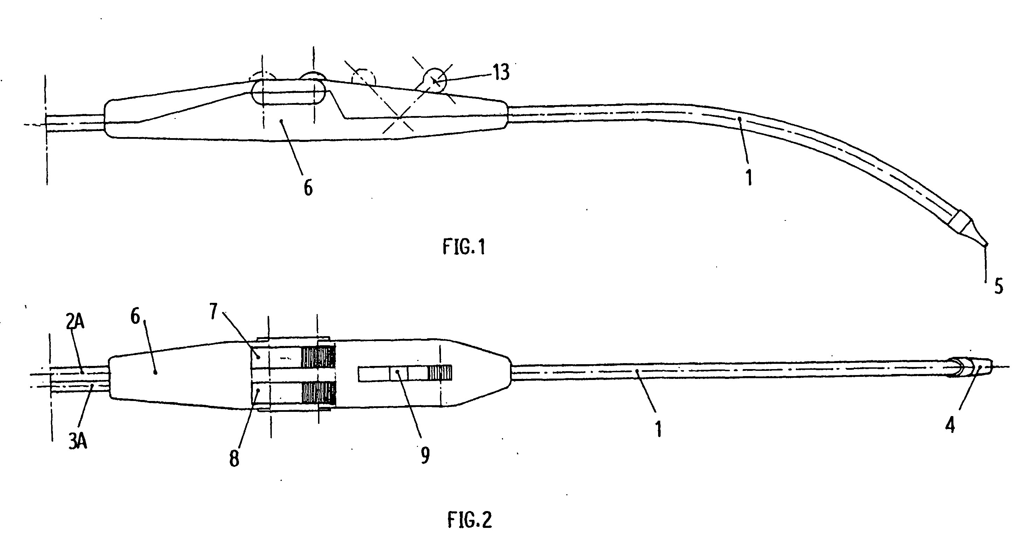

- la figure 1 est une vue de profil du vaporisateur;
- la figure 2 est une vue de dessus de celui-ci;

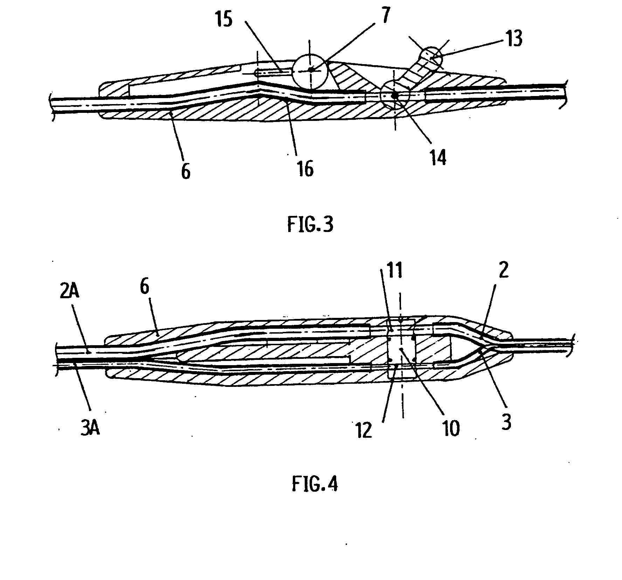

- la figure 3 est une vue en coupe longitudinale du profil de la poignée;
- la figure 4 est une vue en coupe longitudinale de dessus de celle-ci.

Le vaporisateur représenté aux figures comporte essentiellement une canule (1) constituée d'une première canalisation (2) d'amenée d'air comprimé et d'une deuxième canalisation (3) d'amenée de sérum physiologique vers un embout distal (4) servant de chambre de vaporisation et pourvu d'un orifice de sortie (5) du mélange obtenu.

La partie proximale de la canule (1) est reliée aux tuyaux (2A) et (3A) d'amenée de l'air comprimé et du sérum physiologique en provenance d'une source située à distance.

La jonction de la partie proximale de la canule (1), soit les canalisations (2) et (3), et les tuyaux (2A) et (3A) est réalisée dans la zone d'une poignée creuse (6), dans laquelle passent lesdites canalisations et tuyaux, qui comporte deux moyens (7) et (8) à écrasement progressif, notamment du type roulettes de perfuseur, aptes à assurer le réglage, par le chirurgien lui-même, des débits d'air et de sérum.

La poignée (6) comporte, en combinaison, un moyen, du type vanne (9), agissant simultanément sur les passages d'air et de sérum de manière à fermer puis à ouvrir le passage de l'air et du sérum sans modifier les réglages préalablement établis par le chirurgien.

La vanne (9), constituée d'un corps cylindrique rotatif (10) pourvu de deux passages (11) et (12) et d'une poignée d'actionnement (13), comporte un orifice (14) d'évacuation des surpressions d'air.

Les roulettes (7) et (8) se déplacent dans des guides (15) de manière à écraser les tuyaux, ou canalisations, d'air et de sérum, qui reposent dans la zone desdits guides, sur un plan incliné (16).

Les canalisations (2) et (3) sont logées dans un tube souple (1) dans la paroi duquel est intégré un fil métallique malléable apte à maintenir la canule (1) dans la forme désirée.

La chambre de vaporisation de l'embout (4) possède une forme en "bec de canard".

L'orifice de sortie (5) de l'embout (4) qui est réalisé avantageusement en silicone, est ovalisé et aplati de manière à s'ouvrir sous l'effet de la pression du mélange.

Les tuyaux (2A) et (3A) sont collés entre eux longitudinalement jusqu'aux sources d'air comprimé et de sérum.

Dans la version utilisée en chirurgie cardiaque, la poignée (6) et la canule (1) sont réalisées en matière plastique et sont à usage unique.

Dans la version utilisable en chirurgie viscérale, la poignée (6) est réalisée en matériau stérilisable et est réutilisable, la canule (1) est réalisée en matière plastique et est interchangeable et à usage unique et l'ensemble comporte, en addition, un moyen d'aspiration du mélange sang-sérum.

Le moyen d'aspiration, du type tuyau, peut être intégré dans la canule (1) et dans la poignée (6) qui est dans ce cas démontable pour en assurer le nettoyage dans de bonnes conditions.

Les jonctions de tuyaux (2A, 3A) et des canalisations (2,3) peuvent se faire dans la poignée ou à l'extérieur de celle-ci au moyen de connecteurs amovibles.

Dans la réalisation préférée de l'invention :
- l'ensemble canule-poignée a une longueur approximative de 310mm avec 120mm pour la poignée et 190mm pour la canule;
- le tube souple, qui regroupe les deux canalisations de la canule, est en chlorure de polyvinyle de 5mm de diamètre,
- la canalisation de la canule d'amenée de sérum physiologique est en polypropylène, ou polyéthylène, de diamètre 0,5mm;
- la canalisation d'amenée d'air comprimé est en chlorure de polyvinyle de diamètre 5mm.

Bien entendu, l'invention n'est pas limitée aux modes de réalisation décrits et représentés pour lesquels on pourra prévoir d'autres variantes, en particulier dans :
- la nature des matériaux entrant dans la composition des divers sous-ensembles du vaporisateur;
- les dimensions desdits sous-ensembles;
- les types de moyens de réglage du débit et de fermeture des canalisations.

## Revendications

1. Vaporisateur chirurgical de sérum physiologique, destiné à nettoyer le champ opératoire de toute présence de sang gênante pour le chirurgien, du genre comportant une canule (1) pourvue d'une première canalisation (2) d'amenée d'air comprimé et d'une deuxième canalisation (3) d'amenée de sérum physiologique vers un embout distal (4) servant de chambre de vaporisation et pourvu d'un orifice de sortie (5) du mélange obtenu; la partie proximale de ladite canule (1) étant reliée aux tuyaux (2A) et (3A) d'amenée d'air comprimé et de sérum physiologique en provenance d'une source située à distance; la jonction de la partie proximale de ladite canule (1), soit les canalisations (2) et (3), et les tuyaux (2A) et (3A) d'amenée d'air comprimé et de sérum physiologique au vaporisateur proprement dit, étant réalisée dans la zone d'une poignée creuse (6) dans laquelle passent lesdites canalisations qui comportent des moyens permettant le réglage du débit, soit séparément, soit simultanément;
caractérisé en ce ladite poignée (6) comporte, en combinaison, d'une part deux moyens (7) et (8) à écrasement progressif, indépendants, du type à roulette de perfuseur, aptes à assurer le réglage, par le chirurgien lui-même, au cours de l'intervention, des débits d'air et de sérum et d'autre part un moyen (9), du type vanne constitué d'un corps cylindrique rotatif (10) pourvu de deux passages (11) et (12), d'une poignée d'actionnement (13) et d'un orifice (14) d'évacuation des surpressions d'air, apte à agir, simultanément, sur les passages de l'air et du sérum sans modifier les réglages préalablement effectués par le chirurgien par l'intermédiaire desdits moyens (7) et (8).

2. Vaporisateur, selon la revendication 1, **caractérisé en ce que** les moyens (7) et (8) se déplacent dans des guides (15) de manière à écraser les tuyaux, ou canalisations, d'amenée d'air et de sérum, qui reposent, dans la zone desdits guides, sur un plan incliné (16).

3. Vaporisateur, selon la revendication 1, **caractérisé en ce que** les canalisations (2) et (3) sont logées dans un tube souple (1) dans la paroi duquel est intégré un fil métallique malléable apte à maintenir la canule (1) dans la forme désirée.

4. Vaporisateur, selon la revendication 1, **caractérisé en ce que** la chambre de vaporisation de l'embout (4) possède une forme en "bec de canard".

5. Vaporisateur, selon la revendication 1, **caractérisé en ce que** les tuyaux (2A) et (3A) d'amenée d'air et de sérum sont collés entre eux longitudinalement jusqu'aux sources d'air comprimé et de sérum.

6. Vaporisateur, selon l'une quelconque des revendications 1 à 5, **caractérisé en ce que**, dans la version utilisable en chirurgie cardiaque, la poignée (6) et la canule (1) sont réalisées en matière plastique et sont à usage unique.

7. Vaporisateur, selon l'une quelconque des revendications 1 à 5, **caractérisé en ce que**, dans la version utilisable en chirurgie viscérale, la poignée est réalisée en matériau stérilisable et est réutilisable, la canule (1) est réalisée en matière plastique et est interchangeable et à usage unique et l'ensemble comporte, en addition, un moyen d'aspiration du mélange sang-sérum.

8. Vaporisateur, selon la revendication 7, **caractérisé en ce que** le moyen d'aspiration, du type tuyau, est intégré dans la canule (1) et dans la poignée (6).

## Claims

1. Saline surgical sprayer designed to clean out of the operative field any presence of blood disturbing the surgeon of the kind comprising a cannula (1) provided with a first duct (2) for supplying compressed air and a second duct (3) for supplying saline towards a distal nozzle (4) acting as spraying chamber and provided with an outlet (5) for the mixture obtained; the proximal part of the said cannula (1) being connected to the compressed air supply and the saline pipes (2A) and (3A) coming from a source far away; the junction of the proximal part of the said cannula (1), that is to say ducts (2) and (3) and pipes (2A) and (3A) supplying compressed air and saline to the sprayer proper is carried out in the area of a hollow handle (6) in which the said ducts pass, which comprises means allowing to adjust the output, separately or simultaneously;
**characterized in that** the said handle (6) comprises, matched, on one hand independent means (7) and (8) of gradual crushing, of the type of perfusion device castor, adapted for securing the adjustment, by the surgeon himself, during the operation, of the air and saline outputs and, on the other hand, a means (9) gatelike, constituted by a rotatory cylindrical body (10) provided with two passageways (11) and (12), a driving handle (13) and a hole (14) for discharging air pressures, adapted for simultaneously acting on the passages of the air and the saline without modifying previous adjustments carried out by the surgeon through the said means (7) and (8).

2. Sprayer, according to claim 1, **characterized in that** the means (7) and (8) are moving within rails (15) so that they crush the air or saline pipes or ducts which are resting in the area of the said rails on an inclined plane (16).

3. Sprayer according to claim 1, **characterized in that** ducts (2) and (3) are housed within a flexible pipe (1) on the wall of which a malleable metallic wire is integrated adapted to keep the cannula (1) with the shape wished.

4. Sprayer according to claim 1, **characterized in that** the spraying chamber of the nozzle (4) possesses a "duck peak" shape.

5. Sprayer according to claim 1, **characterized in that** pipes (2A) and (3A) supplying air and saline are stuck to each other lengthwise up to the compressed air and saline sources.

6. Sprayer, according to any of claims 1 to 5, **characterized in that**, in the version usable in heart surgery, handle (6) and cannula (1) are made of plastic material and are for a single use.

7. Sprayer, according to any of claims 1 to 5, **characterized in that** in the version usable in visceral surgery, the handle is made of sterilizable material and is reusable, the cannula (1) is made of plastic material and is interchangeable and for a single use and the assembly in addition comprises a means for sucking the blood-saline mixture.

8. Sprayer, according to claim 7, **characterized in that** sucking means, pipe-like, can be integrated within the cannula (1) and within the handle (6).

## Patentansprüche

1. Chirurgischer Zerstäuber für physiologisches Serum, der dazu dient, aus dem Operationsbereich eventuell vorhandenes Blut, das die Arbeit des Chirurgen behindern könnte, zu entfernen, mit einer Kanüle (1) mit einem ersten Kanal (2) zur Zufuhr von Druckluft und einem zweiten Kanal (3) zur Zufur von physiologischem Serum zu einer distalen Düse (4), die als Zerstäubungskammer dient und mit einem Auslass (5) für die resultierende Mischung ausgestattet ist, wobei der proximale Teil der Kanüle (1) mit der von weit her kommenden Druckluftzufuhr und den Röhren (2A) und (3A) für physiologisches Serum verbunden ist; wobei die Verbindungsstelle des proximalen Teils der Kanüle (1), also die Kanäle (2) und (3) sowie die Röhren (2A) und (3A), die den eigentlichen Zerstäuber mit Druckluft und physiologischem Serum versorgen im Bereich eines hohlen Griffs (6) ausgeführt ist, durch den die genannten Kanäle hindurchgeführt werden, wobei ebenfalls Mittel vorhanden sind, mit denen die abgegebene Menge getrennt oder gleichzeitig reguliert werden kann, **dadurch gekennzeichnet, dass** der Griff (6), aufeinander abgestimmt, einerseits unabhängige Mittel (7) und (8) zum allmählichen Zusammendrücken in Art eines Zersprühers mit Rollen, der so eingerichtet ist, dass die Regulierung der Abgabe von Luft und physiologischem Serum während der Operation vom Chirurgen gewährleistet werden kann, und andererseits ein schieberähnliches Mittel (9) aus einem drehbaren zylindrischen Körper (10) mit zwei Durchgängen (11) und (12), einem Antriebsgriff (13) und einer Öffnung (14) zum Druckausgleich aufweist, die so aufeinander abgestimmt sind, dass sie gleichzeitig auf die Durchgänge für Luft und physiologisches Serum einwirken, ohne dass hierdurch die vom Chirurgen mit Hilfe der Mittel (7) und (8) durchgeführten Voreinstellungen geändert werden.

2. Zerstäuber nach Anspruch 1, **dadurch gekennzeichnet, dass** die Mittel (7) und (8) auf Schienen (15) beweglich sind, so dass die Röhren und Kanäle für die Luft und das physiologische Serum, die im Bereich der Schienen auf einen schrägen Ebene (16) ruhen, dadurch zusammengedrückt werden.

3. Zerstäuber nach Anspruch 1, **dadurch gekennzeichnet, dass** die Kanäle (2) und (3) in einem Gummischlauch (1) lagern, in dessen Wand ein schmiedbarer Metalldraht integriert ist, wodurch der Kanüle (1) die gewünschte Form verliehen werden kann.

4. Zerstäuber nach Anspruch 1, **dadurch gekennzeichnet, dass** die Zerstäubungskammer der Düse (4) die Form eines "Entenschnabels" aufweist.

5. Zerstäuber nach Anspruch 1, **dadurch gekennzeichnet, dass** die Röhren (2A) und (3A) zur Zufuhr von Luft und physiologischem Serum in Längsrichtung bis zur Quelle für Druckluft und physiologisches Serum miteinander verklebt sind.

6. Zerstäuber nach einem der Ansprüche 1 bis 5, **dadurch gekennzeichnet, dass** bei der Ausführung für Herzchirurgie der Griff (6) und die Kanüle (1) aus Kunststoffmaterial bestehen und zur einmaligen Verwendung bestimmt sind.

7. Zerstäuber nach einem der Ansprüche 1 bis 5, **dadurch gekennzeichnet, dass** bei der Ausführung für Eingeweidenchirurgie der Griff aus einem sterilisierbaren Material besteht und wiederverwendbar ist, die Kanüle (1) aus Kunststoffmaterial besteht und auswechselbar und zur einmaligen Verwendung bestimmt ist und der Aufbau zusätzlich ein Mittel zum Absaugen der Mischung aus Blut und physiologischem Serum aufweist.

8. Zerstäuber nach Anspruch 7, **dadurch gekennzeichnet, dass** das Absaugmittel röhrenähnlich ist und in die Kanüle (1) und den Griff (6) integriert werden kann.
